# EUROPEAN PATENT SPECIFICATION

(11) **EP 0 701 422 B1**
(45) Date of publication and mention of the grant of the patent: **16.09.1998**
(21) Application number: 94914955.3
(22) Date of filing: 04.05.1994
(51) Int. Cl.: A61F 13/15

(54) **ABSORBENT ARTICLE HAVING TUCKED FLAPS**
ABSOBIERENDER ARTIKEL MIT EINGESCHLAGENEN KLAPPEN
ARTICLE ABSORBANT MUNI DE RABATS REPLIES

(30) Priority: 28.05.1993 US 68916
(43) Date of publication of application: 20.03.1996
(73) Proprietor: THE PROCTER & GAMBLE COMPANY, Cincinnati, Ohio 45202 (US)
(72) Inventor: LAVASH, Bruce, William, West Chester, OH 45069 (US); OSBORN, Thomas, Ward, III, Cincinnati, OH 45224 (US)
(74) Representative: Hirsch, Uwe Thomas
(86) International application number: US9404943
(87) International publication number: WO9427542

(56) References cited:
- WO-A-93/06805
- WO-A-94/00091
- WO-A-94/13237
- US-A- 3 901 239
- US-A- 4 687 478

## Description

### FIELD OF THE INVENTION

The present invention relates to disposable absorbent articles such as female sanitary napkins, adult incontinence devices, and the like. Still more particularly, the present invention concerns such disposable absorbent articles having tucked side flaps.

### BACKGROUND OF THE INVENTION

All manner and variety of absorbent articles configured for the absorption of body fluids such as menses, urine, and feces are, of course, well known. Absorbent articles, particularly sanitary napkins, having wings or flaps are disclosed in the literature and are available in the marketplace.

Generally, the flaps extend laterally from a central absorbent means and are intended to be folded around the edges of the wearer's panties in the crotch region. Thus, the flaps are disposed between the edges of the wearer's panties in the crotch region and the wearer's thighs. Commonly, the flaps are provided with an attachment means for affixing the flaps to the underside of the wearer's panties.

The flaps serve at least two purposes. First, the flaps prevent exudates which otherwise would soil the edges of the wearer's panties from doing such. Second, the flaps help stabilize the napkin from shifting out of place, especially when the flaps are affixed to the underside of the panties.

Sanitary napkins having flaps of the various types are disclosed in U.S. Patent 4,687,478, entitled "Shaped Sanitary Napkin With Flaps", which issued to Van Tilburg on August 18, 1987, U.S. Patent 4,608,047, entitled "Sanitary Napkin Attachment Means", which issued to Mattingly on August 26, 1986, U.S. Patent 4,589,876, entitled "Sanitary Napkin", which issued to Van Tilburg on May 20, 1986, U.S. Patent 4,285,343, entitled "Sanitary Napkin", which issued to McNair on August 25, 1981, U.S. Patent 3,397,697, entitled "Disposable Sanitary Shield For Undergarments", which issued to Rickard on August 20, 1968, and U.S. Patent 2,787,271, entitled "Sanitary Napkin", which issued to Clark on April 2, 1957.

While flaps greatly improve the effectiveness of a sanitary napkin, the flaps of a sanitary napkin may hinder or impede application of the sanitary napkin to the crotch of the wearer's panty. Currently, each of the flaps of a sanitary napkin have an end, the distal end, which may move freely relative to the sanitary napkin. Once the release paper of the central pad adhesive is removed by the wearer, the distal ends of the flaps may fall between the crotch portion of the wearer's panty and the sanitary napkin and may become adhered to the central pad adhesive. Therefore, there is a need for a sanitary napkin having flaps positioned so that they will not interfere with the application of the sanitary napkin to the panty.

Currently, sanitary napkins having flaps will not function properly unless the flaps are used (i.e, are folded down along the edges of the crotch of the wearer's undergarment and affixed to the underside of the undergarment). Generally, as they are packaged, the flaps of a sanitary napkin are folded over the garment side of the sanitary napkin or are folded over the body-facing side of the sanitary napkin. Therefore, if the flaps are not used while the sanitary napkin is being used, the flaps will either obstruct the surface intended to receive bodily exudates on the body-facing side of the sanitary napkin, or will obstruct the adhesive or other fastening means positioned on the garment side of the sanitary napkin. While sanitary napkins having flaps are commonly viewed as providing better protection against soiling as compared to sanitary napkins without flaps, some women still prefer a sanitary napkin without flaps, and some women who generally prefer a sanitary napkin with flaps, occasionally (such as during periods of light flow) prefer a sanitary napkin without flaps. Therefore, there is a need for a sanitary napkin having flaps which may or may not be used while the sanitary napkin is being used.

Accordingly, it is an object of the present invention to provide an absorbent article, such as a sanitary napkin, having tucked side flaps, i.e., flaps which are tucked in a recessed area, which will not impede or hinder application of the absorbent article to the crotch of the wearer's panty.

It is also an object of the present invention to provide an absorbent article, such as a sanitary napkin, having optional side flaps, i.e., flaps which are tucked into a recessed area and which may or may not be used while the absorbent article is being used.

It is an additional object of the present invention to provide an absorbent article having tucked side flaps and zones of differential extensibility for relieving the stresses that develop in the flaps when they are folded down along the edges of the crotch of the wearer's undergarments and affixed to the underside of the undergarments.

These and other objects of the present invention will be more readily apparent when considered in reference to the following description and when taken in conjunction with the accompanying drawings.

### SUMMARY OF THE INVENTION

The present invention is an absorbent article, such as a sanitary napkin, having tucked side flaps which is provided in accordance with claim 1.

The sanitary napkin has a main body portion and a pair of flaps joined to the main body portion at a line of juncture. The main body portion comprises an absorbent assembly and a pair of retaining members joined to the absorbent assembly to form a pair of recessed areas. The absorbent assembly comprises an absorbent core and two spaced apart longitudinal edges. Each of the retaining members comprise an inward longitudinal edge and an outward longitudinal edge. At least a portion of the inward longitudinal edge of each retaining member is joined to the absorbent assembly inboard of the longitudinal edge of the main body portion and the portion of the retaining member that is decoupled from the absorbent assembly forms a recessed area. Each of the flaps has a proximal edge adjacent the line of juncture and a distal edge disposed away from the line of juncture. A portion of one of the flaps is capable of being tucked into one of the recessed areas and a portion of the other flap is capable of being tucked into the other recessed area.

### BRIEF DESCRIPTION OF THE DRAWINGS

Figure 1 is a perspective view of a sanitary napkin embodiment of the present invention.

Figure 1A is a cross-sectional view of the sanitary napkin of Figure 9 taken along section line A-A.

Figure 2 is a perspective view of an alternate sanitary napkin embodiment of the present invention.

Figure 3 is a perspective view of the crotch portion of a women's panties.

Figure 3A is the same perspective view of the women's panties shown in Figure 3 with the sanitary napkin embodiment of the present invention being placed therein for use, with the flaps extended and affixed to the underside of the panties.

Figure 3B is the same perspective view of the women's panties shown in Figure 3 with the sanitary napkin embodiment of the present invention with the flaps tucked into the recessed areas, being placed therein for use.

### DETAILED DESCRIPTION OF PREFERRED EMBODIMENTS

### 1. Introduction

### A. The Absorbent Article In General

The present invention relates to disposable absorbent articles, such as female sanitary napkins. More particularly, the present invention relates to such disposable absorbent articles having flaps that are folded down along the edges of the crotch of the wearer's undergarments and attached to the underside of the undergarments.

The term "absorbent article", as used herein, refers to articles which absorb and contain body exudates. More specifically, the term refers to articles which are placed against or in proximity to the body of the wearer to absorb and contain the various exudates discharged from the body. The term "absorbent article" is intended to include sanitary napkins, pantiliners, and incontinent pads (and other articles worn in the crotch region of a garment). The term "disposable" refers to articles which are intended to be discarded after a single use and preferably recycled, composted, or otherwise disposed of in an environmentally compatible manner. (That is, they are not intended to be laundered or otherwise restored or reused as an absorbent article.) As used herein the terms "optional flaps" or "tucked flaps" shall refer to the flaps of an absorbent article, which are tucked into a recessed area, or are capable of being tucked into a recessed area. A flap is capable of being tucked into a recessed area if it is joined to the sanitary napkin such that at least a portion of the flap may be positioned between the decoupled portion of a retaining member and the absorbent assembly of the main body portion. In the preferred embodiment illustrated, the absorbent article is a sanitary napkin designated 20.

The term "sanitary napkin", as used herein, refers to an article which is worn by females adjacent to the pudendal region that is intended to absorb and contain the various exudates which are discharged from the body (e.g., blood, menses, and urine). The present invention, however, is not limited to the particular types or configurations of absorbent articles shown in the drawings.

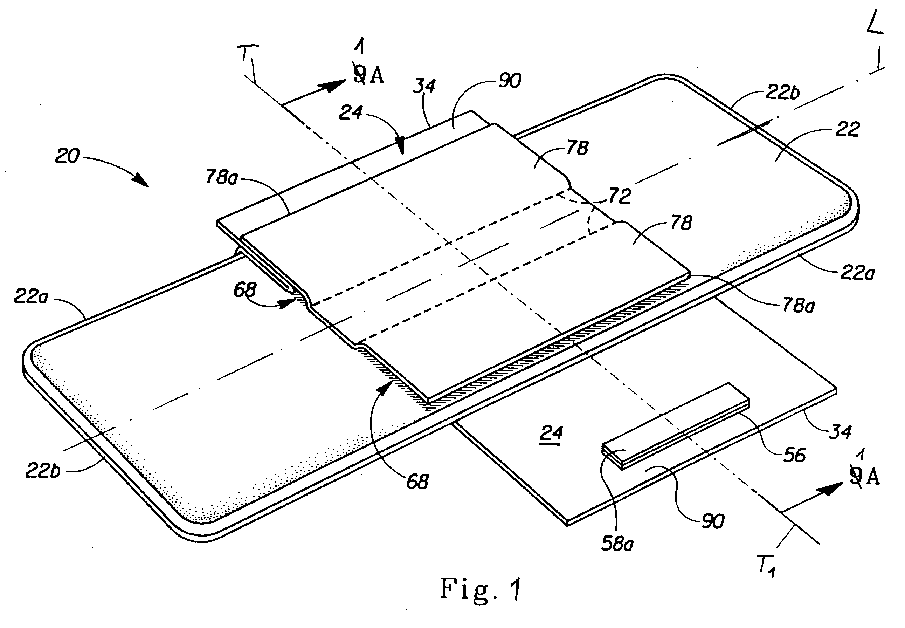

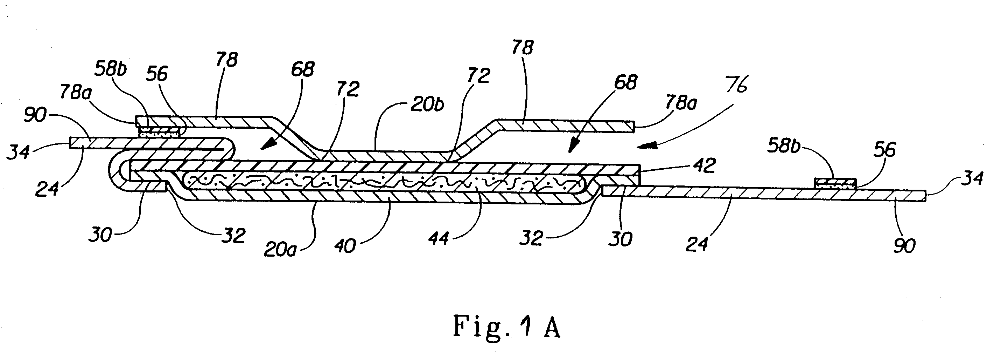

A preferred embodiment of a sanitary napkin 20 of the present invention is shown in Figure 1. As shown in Figure 1, the sanitary napkin 20 basically comprises a main body portion 22 and two flaps 24 (are shown in the extended position) joined to the main body portion 22. The main body portion 22 comprises an absorbent means represented by an absorbent assembly and two retaining members 78 joined to the absorbent assembly. (In the discussion that follows, unless otherwise noted, the sanitary napkin described herein will have two retaining members. While it is not necessary that the sanitary napkin have two retaining members, two retaining members are preferred over one retaining member. Also, while it is not necessary that the retaining members be mirror images of one another, they preferably are. Thus, the description of one retaining member will be a description of the other, and, for clarity, discussion of the second retaining member may be omitted.)

As used herein, the term "joined" encompasses configurations whereby an element is directly secured to the other element by affixing the element directly to the other element; configurations whereby the element is indirectly secured to the other element by affixing the element to intermediate member(s) which in turn are affixed to the other element; and configurations whereby one element is integral with another element, i.e., one element is essentially part of the other element.

The retaining members 78 are each joined to the absorbent assembly at a point of connection 72. As used herein, the term "point of connection" refers to regions where the retaining member 78 is joined to the absorbent assembly of the main body portion 22. These regions can be of any shape or configuration, but they are not limited to spots or points. Thus, these regions can comprise flanges, strips, intermittent lines, spots, and the like. In the embodiment illustrated in Figure 1, each point of connection 72 comprises a linear bond site.

The absorbent assembly preferably comprises a liquid pervious topsheet 40, a liquid impervious backsheet 42 joined to the topsheet 40, and an absorbent core 44 positioned between the topsheet 40 and the backsheet 42.

Both of the flaps 24 shown in Figures 1 and 1A are formed from a discrete piece of material which is attached to the absorbent assembly of the main body portion 22. (In alternative embodiments, such as those shown in U.S. Patent 4,917,697 issued to Osborn, the flaps 24 may be integral with the absorbent assembly of the main body portion 22. In such a case, the topsheet 40 may form one surface of both the flaps 24 and the main body portion 22, and the backsheet 42 may form the other surface of the same. In addition, the absorbent material of the sanitary napkin 20 may extend into the flaps 24 to form a flap absorbent core, as described in greater detail in U.S. Patent 4,917,697. In other alternative embodiments, each flap may be comprised of a separate piece of material joined to the main body portion 22. Such embodiments are shown in PCT Patent Application WO 93/06805, "Absorbent Article Having Flaps and Zones of Differential Extensibility" filed October 1, 1991 in the name of Bruce Lavash, et al. and the respective priority documents. In the discussion that follows, unless otherwise noted, the sanitary napkin described herein will have two flaps. While it is not necessary that the sanitary napkin have two flaps, two flaps are preferred over one flap. Also, while it is not necessary that the flaps be mirror images of one another, they preferably are. Thus, the description of one flap will be a description of the other, and, for clarity, discussion of the second flap may be omitted.

The sanitary napkin 20 has two centerlines, a principal longitudinal centerline L and a principal transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

The flaps 24 are each associated with the main body portion 22 along a juncture 30. This is typically a longitudinally-oriented (or "longitudinal") juncture, such as lines of juncture 30. As used herein, the terms "juncture" (or "line of juncture") refer to regions where the flaps 24 extend from or are joined to the main body portion 22. These regions can be any of various curved or straight lines, but they are not limited to lines. Thus, these regions can comprise flanges, strips, intermittent lines, and the like.

The flaps 24 have a proximal edge 32 adjacent the line of juncture. A distal edge (or "free end") 34 is remote from the line of juncture 30. Each flap 24 is divided into a front half, and a back half by a flap transverse centerline T₁. The flap transverse centerline T₁ may coincide with the principal transverse centerline T of the sanitary napkin (as shown in Fig. 1), but this is not absolutely required.

A sanitary napkin 20 of the present invention comprises at least one recessed area 68 (preferably two recessed areas) in which the flaps 24 may be tucked. The recessed area 68 is formed between the retaining member 78 and the absorbent assembly of the main body portion 22. The recessed area 68 will be formed by joining the the retaining member 78 with the absorbent assembly of the main body portion 22 at points of connection 72. The recessed area 68 will have a mouth 76. The mouth 76 is formed between the longitudinal edge 78a of the decoupled portion of the retaining member 78 and the absorbent assembly of the main body portion 22.

### 2. The Individual Components of the Absorbent Article

The individual components of the main body portion 22 of the sanitary napkin 20 will first be looked at in greater detail.

### A. The Absorbent Assembly

### 1. The Topsheet

The topsheet 40 is liquid permeable and when the sanitary napkin 20 is in use, the topsheet 40 is in close proximity to the skin of the user. The topsheet 40 is compliant, soft feeling, and non-irritating to the user's skin. It can be made from any of the materials conventional for this type of use. Nonlimiting examples of suitable materials that can be used as topsheet 40 are woven and nonwoven polyester, polypropylene, nylon, and rayon and formed thermoplastic films, with formed films being preferred.

Suitable formed films are described in U.S. Patent 3,929,135, entitled "Absorptive Structure Having Tapered Capillaries", which issued to Thompson on December 30, 1975, U.S. Patent 4,324,426, entitled "Disposable Absorbent Article Having A Stain-Resistant Topsheet", which issued to Mullane and Smith on April 13, 1982, U.S. Patent 4,342,314, entitled "Resilient Plastic Web Exhibiting Fiber-Like Properties", which issued to Radel and Thompson on August 3, 1982, and U.S. Patent 4,463,045, entitled "Macroscopically Expanded Three-Dimensional Plastic Web Exhibiting Non-Glossy Visible Surface and Cloth-Like Tactile Impression", which issued to Ahr, Louis, Mullane, and Ouellette on July 31, 1984. Formed films are preferred for topsheet 40 because they are pervious to liquids and yet non-absorbent. Thus, the surface of the formed film which is in contact with the body remains dry and is more comfortable to the wearer.

The sanitary napkin 20 may also be comprised of components that are extensible (i.e., capable of stretching, particularly in the longitudinal direction) when the sanitary napkin is worn. The sanitary napkin 20 may capable of elongating between about 15% and about 40% of its unstretched length. This extensibility provides better in-use fit, comfort, and decreased staining. In other embodiments, only limited portions of the components of the sanitary napkin 20 are capable of stretching. Such an embodiment (without the retaining member of the present invention) is described in greater detail in PCT Patent Application WO 93/06805, "Absorbent Article Having' Flaps and Zones of Differential Extensibility", filed October 1, 1991, in the name of Bruce Lavash, et al.

A particularly preferred topsheet 40 for use in such an embodiment is one which is made in accordance with U.S. Patent 4,463,045 and ring rolled to provide it with a degree of longitudinal extensibility. Suitable processes for ring rolling or "pre-corrugating" are described in U.S. Patent 4,107,364 issued to Sisson on August 15, 1978, U.S. Patent 4,834,741 issued to Sabee on May 30, 1989 and in PCT publication WO 92/15444 "Improved Method And Apparatus For Incrementally Stretching A Zero Strain Stretch Laminate Web To Impart Elasticity Thereto" filed by Gerald M. Weber et al. on February 28, 1991, European Patent Application EP-A-575509 entitled "Improved Method and Apparatus For Incrementally Stretching Zero Strain Stretch Laminate Web In A Non-Uniform Manner To Impart A Varying Degree of Elasticity Thereto" filed by Kenneth B. Buell et al. on February 28, 1991, and European Patent Application EP-A-573587 entitled "Improved Method And Apparatus For Sequentially Stretching Zero Strain Stretch Laminate Web To Impart Elasticity Thereto Without Rupturing The Web" filed by Gerald M. Weber et al. on February 28, 1991. The fold lines in the corrugations of the topsheet should run in the transverse direction so the topsheet is longitudinally extensible.

Such a topsheet is described in greater detail in the following patent applications which were filed on June 23, 1991: PCT Patent Application WO 93/01779 entitled "Absorbent Articles, Especially Catamenials, Having Improved Fluid Directionality, Comfort and Fit" filed in the names of Thompson, et al .; and, PCT Patent Application WO 93/02251 entitled "Absorbent Core for Use in Catamenial Products" filed in the names of Buenger, et al. These patent applications may be referred to collectively as the "Capillary Channel Fiber" patent applications.

In addition, in preferred embodiments of the present invention, at least a portion of the outer surface 40a of the topsheet 40 is treated with a surfactant. It is preferred that the surfactant be substantially evenly and completely distributed across at least the portion of the outer surface of topsheet 40 that overlays the absorbent assembly of the main body portion 22. This can be accomplished by any of the common techniques well known to those skilled in the art. For example, the surfactant can be applied to topsheet 40 by spraying, by padding, or by the use of transfer rolls.

Treating the outer surface of the topsheet 40 with a surfactant renders the surface of the topsheet 40 more hydrophilic. This results in liquid penetrating the topsheet 40 faster than it would if the surface were not treated. This diminishes the likelihood that menstrual fluids will flow off topsheet 40 rather than being absorbed by the absorbent core 44. Preferably, any portions of the topsheet 40 that overlay the flaps 24 are not treated with the surfactant. This will minimize any tendencies fluids may have to spread laterally across the flaps and to come in contact with the wearer's thighs and other parts of the wearer's body.

In preferred embodiments, the inner surface of topsheet 40 is secured in contacting relation with the absorbent core 44. This contacting relationship results in liquid penetrating topsheet 40 faster than if the topsheet 40 were not in contact with absorbent core 44. The topsheet 40 can be maintained in contact with absorbent core 44 by applying adhesive to the inner surface of the topsheet 40. Suitable adhesives useful for this purpose are described in U.S. Patent 4,917,697. The adhesives can be applied by the same methods as the surfactant is applied to the outer surface of the topsheet 40. The retaining member 78 may be formed from the topsheet 40 or from a portion of the topsheet 40. However, it is preferred that the retaining member be formed from a portion of the flaps 24, a portion of the backsheet 42, or from a discrete piece of material.

### 2. The Absorbent Core

The absorbent core 44 is positioned between the topsheet 40 and the backsheet 42. The absorbent core 44 provides the means for absorbing menstrual fluid. The absorbent core 44 need not have an absorbent capacity much greater than the total amount of menstrual fluid anticipated to be absorbed. The absorbent core 44 is generally compressible, conformable, and non-irritating to the user's skin. It can comprise any material used in the art for such purpose. Examples include comminuted wood pulp which is generally referred to as airfelt, creped cellulose wadding, absorbent foams, absorbent sponges, synthetic staple fibers, polymeric fibers, hydrogel-forming polymer gelling agents, peat moss, or any equivalent material or combinations of materials.

Polymeric gelling agents are those materials which, upon contact with fluids (i.e., liquids) such as water or body fluids, imbibe such fluids and thereby form hydrogels. In this manner, fluids discharged into the absorbent core 44 can be acquired and held by the polymeric gelling agent, thereby providing the articles herein with enhanced absorbent capacity and/or improved fluid retention performance.

The polymeric gelling agent which is employed in the absorbent core 44 will generally comprise particles of a substantially water-insoluble, slightly cross-linked, partially neutralized, hydrogel-forming polymer material. The term "particles", as used herein, can refer to particles in any form, such as in the form of pellets, flakes, or fibers. The characteristics of the absorbent core 44 (including, but not limited to the preferred types of polymer materials used therein, and types of methods which can be used for preparing these polymer particles) are described in greater detail in U.S. Patent 5,009,653 issued to Osborn and the patents incorporated by reference in that patent, the disclosures of which are all incorporated by reference herein.

In one preferred embodiment, the absorbent core 44 is a laminate comprised of a layer of superabsorbent polymer material, such as in the form of particles, disposed between two air-laid tissues, first and second tissue layers (or "upper" and "lower" tissue layers). The first and second tissue layers provide containment of the superabsorbent polymer material, improve lateral wicking of the absorbed exudates throughout the absorbent core 44 and provide a degree of absorbency.

A suitable laminate is the superabsorbent laminate WATER-LOCK L-535 available from the Grain Processing Corporation of Muscatine, Iowa (WATER-LOCK registered TM by Grain Processing Corporation). Such superabsorbent laminates are disclosed in U.S. Patent 4,467,012, entitled "Composition For Absorbent Film And Method of Preparation", which issued to Pedersen et al. on August 21, 1984, and U.S. Patent 4,260,443, entitled "Laminated Absorbent Process", which issued to Lindsay et al. on April 7, 1981.

The absorbent core 44 may be a laminate, as described above, which is slitted or partially slitted for longitudinal extensibility. This slitted or partially slitted core is described in greater detail in the Capillary Channel Fiber patent applications.

### 3. The Backsheet

The backsheet 42 is impervious to liquids and, thus, prevents menstrual fluid from soiling the clothing of the user. Any material used in the art for such purpose can be utilized herein. Suitable materials include embossed or nonembossed polyethylene films and laminated tissue. A suitable polyethylene film is manufactured by Monsanto Chemical Corporation and marketed in the trade as Film No. 8020.

In one alternative embodiment of the sanitary napkin 20 (typically in which the topsheet 40 overlays only the main body portion 22 and does not extend out to form the top surface of the flaps), the backsheet 42 may be comprised of two layers. In such a case, the backsheet 42 may comprise a first layer of lofted material disposed on the core-facing side 42a of the backsheet. The purpose of the first layer is to provide a comfortable, non-irritating surface against the body of the wearer. The lofted layer may be comprised of any suitable material, such as a nonwoven material. Preferably, the lofted layer comprises a hydrophobic nonwoven material. The second layer may be disposed on the garment side of the backsheet 42, and may comprise a fluid impervious film. A low density polyethylene material about 0.01 to about 0.05 millimeters in thickness, preferably about 0.02 millimeters in thickness, has been found to work well as this second layer. A polyethylene film, such as is sold by the Ethyl Corporation, Visqueen Division, under model XP-39385 has been found particularly well suited for this second layer. The backsheet 42 may also be made of a soft, cloth-like material which is hydrophobic relative to the topsheet 40. A polyester or polyolefinic fiber backsheet 42 has been found to work well. A particularly preferred soft, cloth-like backsheet 42 material is a laminate of a polyester nonwoven material and a film such as described in U.S. Patent 4,476,180 issued to Wnuk on October 9, 1984.

A particularly preferred extensible backsheet 42 is an extended adhesive film Formula #198-338 manufactured by the Findley Adhesives Company of Wauwatosa, Wisconsin which is described in greater detail in the Capillary Channel Fiber patent applications.

### B. The Retaining Member

The main body portion 22 of the sanitary napkin 20 will have at least one retaining member 78 joined to the absorbent assembly. The retaining member 78 will be joined to the absorbent assembly at a point of connection 72. A recessed area 68 is formed between the retaining member 78 and the absorbent assembly.

The retaining member 78 can be joined to the absorbent assembly of the main body portion 22 in a number of different manners. Many of the different ways a component (such as the retaining member 78) can be "joined to" or "associated with", etc. another component are set forth in the definitions of these terms contained in U.S. Patent 5,007,906 entitled "Decoupled Sanitary Napkin" which issued to Osborn, et al. on April 16, 1991. When the retaining member is comprised of an element discrete from the absorbent assembly i.e. is not integral with the topsheet, backsheet, etc, it can be joined to the absorbent assembly by any techniques known to those skilled in the art. Such techniques include, but are not limited to adhesives, heat and/or pressure, ultrasonics, etc. The point of connection 72 are not limited to points or spots and may comprise flanges, strips, intermittent lines, spots, and the like, or may comprise combinations of flanges, strips, intermittent lines, spots, and the like. Therefore, the point of connection 72 may be a line which is concave, straight, or convex and may form any angle relative to the principal longitudinal centerline L.

The retaining members 78 can each be joined to the absorbent assembly of the main body portion 22 along the principal longitudinal centerline L, or at any place between the principal longitudinal centerline L and the longitudinal edges of the absorbent assembly. The retaining members 78 will, of course, generally be on opposite sides of the principal longitudinal centerline L.

The retaining member 78 is generally longitudinally oriented on the absorbent assembly. The retaining member is longitudinally oriented on the absorbent assembly when the longitudinal edge 78a of the retaining member 78 is oriented in a direction substantially parallel to the longitudinal centerline L or in a direction having a vector component substantially parallel to the longitudinal centerline L.

The retaining member 78 is generally compliant soft feeling and non-irritating to the users skin. The retaining member 78 is preferably made from any of the materials conventionally used for sanitary napkins 20. Examples of suitable materials that can be used for the retaining member 78 are woven and nonwoven polyester, polypropylene, nylon, and polyethylene, as well as plastic films. The retaining member 78 may be comprised of one or more of the elements of the absorbent assembly e.g., topsheet 40, backsheet 42, etc. Preferably, the retaining member 78 will comprise a piece of material discrete from the topsheet, backsheet, etc.

The overall size and shape of the retaining members 78 may be readily selected by those skilled in the art and will be dependent upon the desired size and shape of the recessed area 68 and the size and shape of the flaps 24 as they are tucked into the recessed area 68. Although it is not necessary that the retaining members 78 be mirror images of each other, it is preferred that the retaining members 78 are mirror images of each another. Whether or not the retaining members 78 are symmetrical about the principal transverse centerline T is also dependent upon the desired size and shape of the recessed area 68, as well as the location and symmetry of the flaps 24. However, it should be understood that the retaining members 78 need not have a shape, size, or location which exactly corresponds to the size, shape, and location of the flaps 24. It is only required that the retaining members 78 be sized, shaped, and positioned such that the retaining member 78 forms a recessed area 68 which can accommodate the flap 24 in a tucked configuration.

The recessed area 68 may be much smaller in size and shape than the flaps 24. For example, although the size and shape of the recessed areas 68 of Figure 1 are smaller in size than the flaps 24, it can be seen from Figures 1 and 1A that the recessed areas 68 are sufficient in size and shape to accommodate the flaps 24 in their tucked configuration.

It is not necessary that each retaining member 78 be formed of a separate piece of material. Each retaining member 78 may each be formed from the same piece of material. It is also not necessary that the retaining members 78 be joined to the backsheet 42 of the absorbent assembly 46. The retaining members 78 may be joined to any element of the absorbent assembly 46. Many various configurations of a sanitary napkin 20 having a retaining member 78 joined thereto to form a recessed area 68, will be readily apparent to one skilled in the art.

### 3. Assembly of Components into a Sanitary Napkin and Formation of the Flaps

### A. Assembly of Components

As shown in Figures 1 and 1A, the topsheet 40 is secured to backsheet 42 along a seam. The seam can be formed by any means commonly used in the art for this purpose such as by gluing, crimping, or heat-sealing. The seam can extend completely around the periphery of the absorbent assembly of the main body portion 22. This is a preferred embodiment for ease of construction. (Other means of uniting the various elements can be used.)

The absorbent assembly is the portion of the main body portion 22 that contains an absorbent means, such as absorbent core 44. The absorbent assembly of the main body portion 22 has a liquid pervious body contacting surface (represented in Figure 1A by topsheet 40) and an opposed liquid impervious surface (represented in Figure 1A by backsheet 42). It is to be understood that the embodiment illustrated is only one possible embodiment, albeit a preferred one. Other possible embodiments include one in which an absorbent core 44 is essentially completely wrapped with topsheet before it is placed on a backsheet. The absorbent assembly of the main body portion 22 can also comprise an absorbent core which possesses sufficient integrity to stand alone and is liquid pervious on one surface while the other surface has been treated to render it liquid impervious.

The absorbent assembly of the main body portion 22 may be relatively thick or relatively narrow and thin. A narrow absorbent assembly may be effective because the overall configuration and use of sanitary napkin 20 results in absorbent assembly of the main body portion 22 being maintained in close proximity to the body. Such proximity of the absorbent assembly places it precisely where it should be: very near the body at the vaginal opening. The absorbent assembly of the main body portion 22 can then absorb the vast majority of the menstrual fluid (menses) before it has an opportunity to flow along the sides of the main body portion 22. A thin absorbent assembly may also be desired because it is typically comfortable to the user.

Figures 1 and 1A also show the the flap securement member, flap adhesive 56, which are adapted to secure the sanitary napkin 20 to the crotch region of an undergarment. This can further be supported by a pad securment member, such as a central pad adhesive.

Although the pad securement member is described herein as a central pad adhesive and the flap securement member is described herein as a flap adhesive it should be understood that fastening means other than adhesives can be used as the pad securement member and the flap securement member. Any type of fastener or combination of fasteners used in the art can be used for such a purpose. For example, the sanitary napkin 20 could be secured to the wearer's undergarment by the fastener described in U.S. Patent 4,946,527 entitled "Pressure-Sensitive Adhesive Fastener and Method of Making the Same" issued to Battrell on August 7, 1990. Other examples of fastening means would include mechanical fasteners such as hook and loop type mechanical fasteners or any other mechanical fasteners which are well known in the art. Preferred mechanical fasteners are disclosed in European Patent Application EP-A-603189, "Screen Printing Method for Manufacturing a Refastenable Mechanical Fastening System and Fastening System Produced Therefrom", filed June 21, 1991, in the name of Dennis A. Thomas and David J. K. Goulait, European Patent Application EP-A-509040, "Method for Manufacturing a Refastenable Mechanical Fastening System having azimuthally angled Prongs and Fastening System Produced Therefrom", filed June 21, 1991, in the name of Dennis A. Thomas and David J. K. Goulait, and commonly-assigned, U.S. Patent US-A-5,392,498, "Non-abrasive Mechanical Fastening System and Process of Manufacture Therefor", filed December 10, 1992 in the names of David J. K. Goulait and Dennis A. Thomas. Particularly preferred mechanical fasteners for use on an absorbent article such as a sanitary napkin, are disclosed in commonly-assigned, U.S. Patent 5,300,058, "Disposable Absorbent Article Having An Improved Mechanical Fastening System", filed December 10, 1992 in the names of David J. K. Goulait, Dennis A. Thomas, and Maureen E. Stanley. For simplicity, however, the pad securement member and the flap securement members will be described in terms of adhesive attachment means, i.e., central pad adhesive and flap adhesive 56.

The central pad adhesive provides an adhesive attachment means for securing main body portion 22 in the crotch portion of a panty. The outer surface of flap 24, adjacent the distal edge 34 of the flap, is preferably coated with a flap adhesive 56. The flap adhesive 56 is used to assist in maintaining the flap 24 in position after it is wrapped around the edge of the crotch portion of the panty as described below. The flaps 24 can be maintained in position by attaching the flaps 24 to the undergarment, or to the opposing flap. Suitable adhesive fasteners are described in greater detail in U.S. Patent 4,917,697.

The adhesive attachment means are respectively covered by removable release liners, central pad release liner and flap release liner 58a. The pressure-sensitive adhesives should be covered with release liners to protect the adhesives from dirt, to keep the adhesives from drying out, and to keep the adhesives from sticking to extraneous surfaces prior to use. Suitable release liners are described in U.S. Patent 4,917,697.

While a preferred sanitary napkin embodiment of the present invention has been described, numerous other sanitary napkin embodiments having flaps are available and are disclosed in the literature. These could be provided with the retaining member 78 of the present invention. In particular, sanitary napkins having flaps are disclosed in U.S. Patent US-A-5,346,486 entitled "Sanitary Napkin Having Laterally Extensible Means for Attachment to the Undergarment of the Wearer", filed May 21, 1991 in the name of Osborn, et al.; U.S. Patents 5,009,653 and 4,950,264, both entitled "Thin, Flexible Sanitary Napkin" which issued to Osborn on April 23, 1991 and August 21, 1990, respectively, U.S. Patent 4,940,462, entitled "Sanitary Napkin With Expandable Flaps" which issued to Salerno on July 10, 1990, U.S. Patent 4,917,697 entitled "Sanitary Napkin Having Flaps and Stress Relief Means" which issued to Osborn, III, et al. on April 17, 1990, U.S. Patent 4,911,701, entitled "Sanitary Napkin Having Elastic Shaping Means" which issued to Mavinkurve on March 27, 1990, U.S. Patent 4,900,320, entitled "Sanitary Napkin With Panty Gathering Flaps" which issued to McCoy on February 13, 1990, U.S. Patent 4,687,478, entitled "Shaped Sanitary Napkin With Flaps", which issued to Van Tilburg on August 18, 1987, U.S. Patent 4,608,047, entitled "Sanitary Napkin Attachment Means", which issued to Mattingly on August 26, 1986, U.S. Patent 4,589,876, entitled "Sanitary Napkin", which issued to Van Tilburg on May 20, 1986, U.S. Patent 4,285,343, entitled "Sanitary Napkin", which issued to McNair on August 25, 1981, U.S. Patent 3,397,697, entitled "Disposable Sanitary Shield For Undergarments", which issued to Rickard on August 20, 1968, and U.S. Patent 2,787,241, entitled "Sanitary Napkin", which issued to Clark on April 2, 1957.

Suitable absorbent articles in the form of pantiliners are disclosed in U.S. Patent 4,738,676 entitled "Pantiliner" issued to Osborn on April 19, 1988. Suitable absorbent articles, at least some of which are in the form of adult incontinence products, are described in European Patent Application EP-A-565630 entitled "Absorbent Article Having Rapid Acquiring Wrapped Multiple Layer Absorbent Body" filed by Barry R. Feist, et al. on January 3, 1991.

In an alternative embodiment, the sanitary napkin 20 could be provided with an elastomer, such as an elastomeric strand, elastomeric ribbon, elastomeric film or the like. In such an embodiment the main body portion 22 would preferably comprise such an elastomer joined to at least a portion of each longitudinal edge 22a. A sanitary napkin comprising an elastomer is disclosed in U.S. Patent US-A-5,234,422 "Elasticized Sanitary Napkin", filed December 20, 1991, in the name of Diane Sneller, June Brennock, and Carl Bergman.

### B. Side Flaps

The characteristics of the flaps 24 will now be looked at in greater detail. The general construction of flaps 24 suitable for use in the present invention is described in greater detail in the such as U.S Patent 4,917,697 issued to Osborn; PCT Patent Application WO 93/06805, "Absorbent Article Having Flaps and Zones of Differential Extensibility", filed October 1, 1991 in the name of Bruce Lavash, et al. and it's priority documents Pleated Flaps", filed February 7, 1992 in the name of Kaoru Niihara and Thomas W. Osborn, III.

The overall size of the flaps 24 can be readily selected by those skilled in the art. Preferably, the flaps 24 are sized so that the sanitary napkin 20 is from about 10 to about 23 centimeters wide between the distal edges 34 of the flaps at their greatest separation. Preferably each flap 24 is from about 5 to at least about 19 centimeters long in the direction parallel to the principal longitudinal centerline L of the sanitary napkin. However, the flap 24 may be as small as 0.5 centimeters long in the direction parallel to the principle longitudinal centerline L of the sanitary napkin 20.

The shape of the flaps 24 can be selected by those skilled in the art. Preferably, not only are the flaps 24 mirror images of each other, the two halves of each flap and are also symmetrical about the flap transverse centerline T₁. (It should be understood that the shape and orientation of the flaps described herein are those of a preferred embodiment. They are not mandatory design features.)

The flaps 24 can be associated with the main body portion 22 in a number of different manners. Many of the different ways a component (such as the flaps 24) can be "joined to" or "associated with", etc. another component are set forth in the definitions of these terms contained in U.S. Patent 5,007,906 entitled "Decoupled Sanitary Napkin" which issued to Osborn, et al. on April 16, 1991. When the flaps comprise separate elements, they can be joined to the main body portion 22 by any techniques known to those skilled in the art. Such techniques include, but are not limited to adhesives, heat and/or pressure, ultrasonics, etc.

Each flap 24 is joined to the main body portion 22 and is preferably joined to the longitudinal edge of the absorbent assembly.

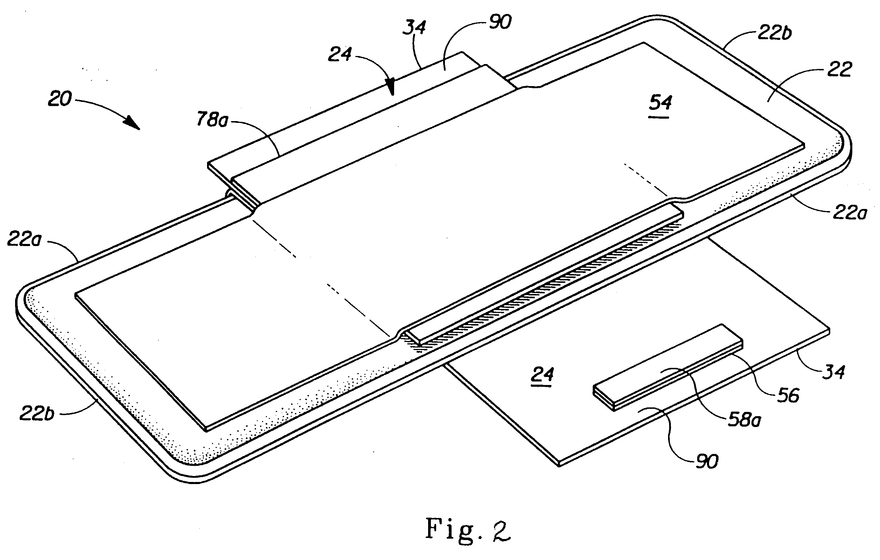

The flaps 24 are joined to the main body portion 22 along lines of juncture 30. The lines of juncture can be concave, straight, (or, but preferably not convex) relative to the principal longitudinal centerline L. The lines of juncture 30 may comprise those lines or areas where separate flap elements are joined to the main body portion 24. Alternatively, when the flaps 24 are integral with the main body portion 22, as in Figures 2 and 2A, the lines of juncture 30 may represent lines of demarcation between the main body portion 22 and the flaps 24 (although it is not necessary that there be a precise line of demarcation).

It is not necessary that the flaps 24 extend from (or be joined along) the longitudinal edges 22a of the main body portion 22. The flaps 24 can joined inward (or "inboard") from the longitudinal edges 22a toward the longitudinal centerline such as is shown in U.S. Patent 4,900,320 issued to McCoy on February 13, 1990. The flaps 24 can, thus, each be joined to the main body portion 22 along the principal longitudinal centerline L, or along the longitudinal edges 22a of the main body portion 22, or at any place between the principal longitudinal centerline L and the longitudinal edges 22a of the main body portion 22. The flaps 24 will, of course, generally be on opposite sides of the principal longitudinal centerline L.

### C. The Recessed Areas

Preferably each flap 24 will have at least one recessed area 68 one on each side of the longitudinal centerline L. The retaining members 78 are joined to the absorbent assembly of the main body portion 22 at the points of connection 72. The point of connection 72 of each retaining member 78 comprises a straight line bond.

It is not necessary that both retaining members 78 be formed of a single piece of material as shown in Figures 1 and 1A. Each retaining member 78 may each be formed from a separate piece of material. It is also not necessary that the retaining members 78 be joined to the backsheet 42 of the absorbent assembly. The retaining members 78 may be joined to any element of the absorbent assembly.

Preferably each tucked flap 24 will be provided with a graspable tab member 90. As used herein, the term "tab member" will refer to an element or component of the sanitary napkin 20 which protrudes form the recessed area 68 and may be used to remove the flap 24 from the recessed area 68. The graspable tab member 90 may extend laterally beyond the longitudinal edge of the absorbent assembly or may extend laterally beyond the longitudinal edge 78a of the retaining member 78. Preferably, the graspable tab member 90 extends laterally beyond the longitudinal edges of both the absorbent assembly and the retaining member 78. The graspable tab member 90 preferably extends from the recessed area 68 at least between about 2 millimeters to about 5 millimeters. More preferably, the tab member 90 extends from the recessed area 68 between about 5 millimeters to about 10 millimeters. A preferred tab member 90 is formed by folding, pleating, or corrugating the flap 24 such that the distal edge 34 of the flap 24 protrudes from the mouth 76 of the recessed area 68. There are many different fold configurations which will result in the distal edge 34 of the flap 24 protruding from the mouth 76 of the recessed area 68. An example of particularly preferred fold configurations which results in the distal edge of the flap 24 forming a tab member 90, is shown in Figure 1A. Other suitable fold configurations will be readily apparent to those skilled in the art.

The flap adhesives 56 can be provided with a release liner 58b. The release liner can have a release portion and a tab portion. The release portion is removably secure to the flap adhesive 56 and the tab portion can extend from the mouth 76 of the recessed area 68 when the flap 24 is tucked into the recessed area 68. Pulling upon the tab portion of the release liner 58b, causes the flap 24 to be drawn out of the recessed area 68. Pulling upon the tab portion of the release liner 58b, preferably also causes the flap adhesive 56 to separate from the release portion of the release liner 58b. If present the tab portion is generally compliant soft feeling and non-irritating to the users skin. The tab portion is preferably made from any of the materials conventionally used for sanitary napkins 20. Preferably the tab portion 84 will be comprised of the same material as the release liner 58b. In a preferred embodiment the tab portion will simply be an extension of the release liner 58b.

Although release liners are generally not used with mechanical fastening materials, a mechanical fastening material may be provided with a release liner. If a flap securement member comprising a mechanical fastener (not shown) is tucked into the recessed area such that the distal edge of the flap is positioned in the recessed area and will not form a tab member, it is preferred that the mechanical fastening material of the securement member be provided with a release liner comprising a tab portion and a release portion. The release portion will be removably secured to the mechanical fastening material and at least a portion of the tab portion will extend outside of the recessed area to form a tab member. The release portion will preferably comprise a fibrous material such as the loop fastening materials which are well known in the art. The tab portion will comprise any material which is compliant, soft, non-irritating to the skin, and which is strong enough to pull the flap out of the recessed are without breaking, rupturing, etc. Preferably the tab portion will be comprised of those materials commonly used for sanitary napkins.

In a particularly preferred sanitary napkin embodiment of the present invention one longitudinal edge of the retaining member will be joined to the absorbent assembly at a point of connection inboard of the longitudinal edge 22a of the main body portion 22.

It should be noted that a sanitary napkin will generally also comprise a central pad adhesive for affixing the main body portion 22 to the wearer's panty. Any of the embodiments shown and described herein may also be provided with a central pad adhesive. However, the central pad adhesive has been omitted from the drawing figures for clarity.

Figure 1 is a perspective view of another sanitary napkin embodiment of the present invention. Figure 1A is a cross-sectional view of the sanitary napkin of Figure 1 taken along section line A-A. It can be seen that the flaps 24 of the sanitary napkin 20 are joined to the absorbent assembly of the main body portion 22. The discrete flaps 24 are affixed to the longitudinal edge 22a of the absorbent assembly. The retaining members 78 are joined to the absorbent assembly at a point of connection 72 inboard of the longitudinal edge 22a of the absorbent assembly forming a recessed area 68 wherein the flaps 24 can be stored or tucked.

Although both of the retaining members 78 are formed from a single piece of material in the embodiments shown in Figures 1 1a, it is not necessary that both retaining members be formed from a single piece of material. Each retaining member 78 may be formed from a discrete piece of material. It is also not necessary that the retaining members be discrete from the absorbent assembly. Each retaining member 78 may be integral with the absorbent assembly, e.g. formed from a portion of the absorbent assembly such as the backsheet 42, topsheet 40, etc.

It is preferred that the retaining members 78 be formed from a material that is sufficiently rigid to maintain its shape such that the retaining member will hold the flap in a tucked position. Suitable retaining members 78 may be manufactured from a wide range of materials such as woven and non-woven materials; and polymeric materials such as thermoplastic films, apertured formed thermoplastic films, apertured plastic films, and hydroformed thermoplastic films. Suitable woven and non-woven materials can be comprised of natural fibers (e.g., wood or cotton fibers), synthetic fibers (e.g., polymeric fibers such as polyester, polypropylene, or polyethylene fibers) or from a combination of natural and synthetic fibers.

A particularly preferred retaining member 78 comprises an apertured formed film bonded to a layer of polyethylene. The apertured formed film may be bonded to the layer of polyethylene by any means well known in the art such as ultrasonics, adhesives, heat seals, pressure bonding, or a combination of heat and pressure bonds. Preferably, apertured formed film is bonded to a layer of polyethylene by bonds formed from a combination of heat and pressure. Methods and apparatus for forming particularly preferred bonds which use a combination of heat and pressure, are disclosed in U.S. Patent 4,919,738, "Dynamic Mechanical Bonding Method And Apparatus", issued April 24, 1990 to Ball et al. and U.S. Patent 4,854,984, "Dynamic Mechanical Bonding Method And Apparatus", issued August 8,1989 to Ball et al.,. The stiffness of the retaining member 78 may be enhanced by using a cross hatched bonding pattern.

Suitable formed films are described in U.S. Patent 3,929,135, issued to Thompson on December 30, 1975; U.S. Patent 4,324,246, issued to Mullane, et al. on April 13, 1982; U.S. Patent 4,342,314, issued to. Radel, et al. on August 3, 1982; U.S. Patent 4,463,045 issued to Ahr, et al. on July 31, 1984; U.S. Patent 4,629,643 issued to Curro, et al. on December 16, 1986; and U.S. Patent 5,006,394 issued to Baird on April 9, 1991. A preferred formed film for use as the retaining member of the present invention or for use as one or more layers of the retaining member of the present invention is formed film described in one or more of the above patents and marketed on sanitary napkins by The Procter & Gamble Company as "Dri-weave".

The retaining member 78 should be sufficiently stiff to hold the flaps 24 in a tucked configuration when the sanitary napkin 20 is suspended with the body-facing side facing upward. When the sanitary napkin is looked at in a cross-sectional view taken along about the flap transverse centerline T₁, the retaining members 78 will form an angle relative to the horizontal plane of the main body portion 22. The angle of the retaining members 78 relative to the horizontal plane of the main body portion 22 is referred to as the support angle S. As used herein, the term "horizontal plane of the main body portion" or simply "horizontal plane" will refer to an imaginary plane which is substantially parallel to the longitudinal centerline L and the transverse centerline T of the main body portion.

When the sanitary napkin 20 is suspended with the body-facing side facing upward the angle of support S will preferably be 45° or less. In a most preferred embodiment, the retaining member will be substantially parallel to the horizontal plane to form a support angle S of about 0°.

If it is desired or necessary to use less rigid materials for the retaining member 78, then the retaining member 78 may be tacked to the flap 24 or the flap 24 may be tacked or releasably bonded to the absorbent assembly to provide additional rigidity.

Referring to Figure 2, there is shown a sanitary napkin embodiment of the present invention comprising a central pad adhesive 54 which is joined to a portion of the backsheet 42 of the absorbent assembly and a portion of the retaining members 78. The central pad adhesive 54 provides additional rigidity to the retaining members 78 and helps to temporarily maintain the retaining members 78 in a horizontal position (i.e., substantially parallel to the horizontal plane of the main body portion). The central pad adhesive 54 is located so as to bridge between the retaining member 78 and the absorbent assembly at a point outboard of the point of connection 72 such that at least a portion of the central pad adhesive is joined to at least a portion of the retaining members 78. The central pad adhesive 54 preferably has sufficient integrity to, at least temporarily, hold the retaining members 78 in a substantially horizontal position while the sanitary napkin 20 is being applied to the panty.

Although the flaps and retaining members shown in several of the drawing figures are depicted as being substantially rectangular in shape, it should be understood that the retaining members 78 and flaps 24 may have any suitable shape.

### 2. Function of the Sanitary Napkin With Respect to an Undergarment

The function of the sanitary napkin of the present invention will now be described in greater detail with relation to the wearer's undergarments.

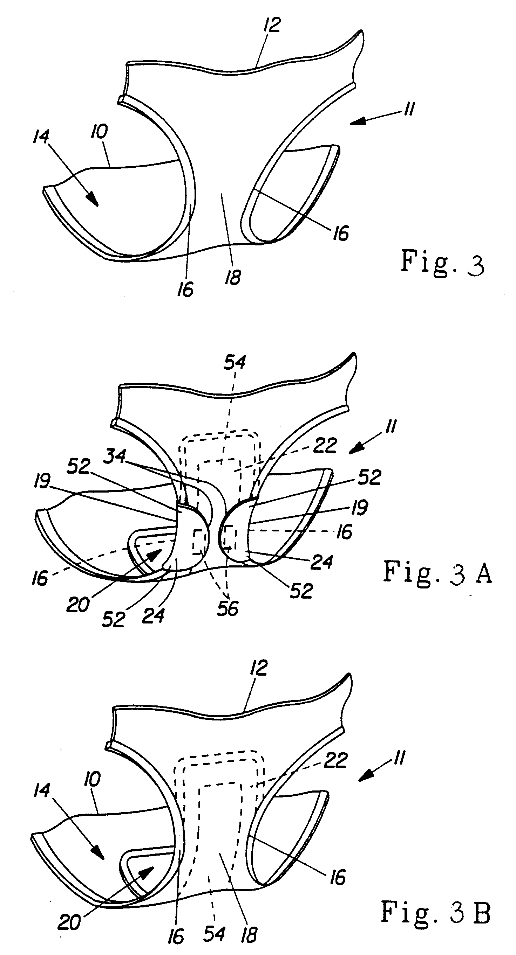

Figure 3 is a depiction of the crotch portion 14 of an undergarment 11 of the type commonly worn by many women and well known as a panty. A panty 11 comprises a front section 10, a back section 12, and a crotch portion 14 which joins the front and back sections. The crotch portion 14 comprises two side edges 16 and center crotch portion 18.

The sanitary napkin 20 of the present invention may be utilized by removing the release liner of the central pad adhesive 54 and placing the sanitary napkin 20 in a panty 11 as shown in Figure 3B. The center of main body portion 22 is placed in crotch portion 14 of the panty with one end of main body portion 22 extending towards the front section 10 of the panty and the other end towards the back section 12. The backsheet 42 is placed in contact with the inner surface of center crotch portion 18 of the panty. Central pad adhesive 54 maintains main body portion 22 in position. The flaps 24 remain in the stored position i.e., tucked in the recessed areas. The panty is pulled up into position on the wearer's lower torso. Although, the flaps 24 have not been used and remain tucked into the recessed areas 68, the flaps 24 will not adversely effect the functionality of the sanitary napkin 20.

Alternatively, the sanitary napkin 20 of the present invention may be utilized by removing the release liner from the central pad adhesive and placing the sanitary napkin 20 in a panty 11 as shown in Figure 3A. The center of main body portion 22 is placed in crotch portion 14 of the panty with one end of main body portion 22 extending towards the front section 10 of the panty and the other end towards the back section 12. The backsheet 42 is placed in contact with the inner surface of center crotch portion 18 of the panty. Central pad adhesive 54 maintains main body portion 22 in position. The flaps 24 are removed from the recessed area 68. The release liners 58a are removed from the flap adhesives 56. The distal portions of flaps 24 are folded around the side edges 16 of the panty, and the flap adhesives 56 are secured to the underside of the panty. In an alternative and most preferred case the sanitary napkin will have at least one zone of differential extensibility, preferably, four zones of differential extensibility, one in each quarter of the sanitary napkin 20.

## Claims

1. An absorbent article (20) for wearing in a user's undergarment (11), said absorbent article (20) having a body-facing side and a garment side, said absorbent article (20) comprising a main body portion (22), a first flap (24), and a second (24), said main body portion (22) having a longitudinal centerline L said main body portion (22) comprising an absorbent assembly having a first longitudinal edge, a second longitudinal edge, said absorbent article (22) being characterized in that it further comprises:
a first and a second retaining member (78) having an inward longitudinal edge and an outward longitudinal edge (78a), said inward longitudinal edge being joined to said garment side of said absorbent article (20), said retaining members (78) being joined at points of connection (72) inboard of the longitudinal edge of said absorbent assembly to form recessed areas (68), said points of connection (72) extending along said inward longitudinal edge of said retaining members
said first and second flap (24) extend laterally outward from said main body portion (22), wherein said flaps (24) have a proximal edge (32) adjacent a line of juncture (30) where they are joined to said main body portion (22) and a distal edge (34) disposed away from the line of juncture (30), and said flaps comprise a flap securement member (56) joined thereto, and at least a portion of said flaps are tucked into said recessed area (68);

2. The absorbent article (20) of Claim 1 wherein said point of connection (72) of said retaining member (78) is (one quarter inch to one inch) 0.6 cm to 2.5 cm, preferably (three quarters of an inch) 2 cm, inward of said longitudinal edge of said absorbent assembly.

3. The absorbent article (20) of Claim 1 wherein said flap (24) comprises a graspable tab member (90) which extends laterally outward beyond the outward longitudinal edge (78a) of said retaining member (78).

4. The absorbent article (20) of Claim 1 wherein said flap (24) is joined to said main body portion (22).

5. The absorbent article (20) of Claim 1 further comprising a central pad securement member that comprises an adhesive which is joined to at least a portion of said retaining members (78).

6. The absorbent article (20) of Claim 1 further comprising a central pad securement member that comprises a mechanical fastening system.

7. The absorbent article (20) of Claim 1 wherein said first flap (24) and said second flap (24) each comprise a flap transverse centerline (T₁) that intersects the principal longitudinal centerline (L) of the absorbent article (20) and divides the absorbent article (20) into four quarter, each of said quarters comprising a zone of differential extensibility.

## Patentansprüche

1. Ein absorbierender Artikel (20) zum Tragen in einem Unterwäschestück (11) eines Verwenders, wobei der genannte absorbierende Artikel (20) eine dem Körper zugewandte Seite und eine Kleidungsstückseite aufweist, der genannte absorbierende Artikel (20) einen Hauptkörperabschnitt (22), einen ersten Lappen (24) und einen zweiten Lappen (24) aufweist, wobei der genannte Hauptkörperabschnitt (22) eine Längsmittellinie (L) aufweist, wobei der genannte Hauptkörperabschnitt (22) einen absorbierenden Aufbau mit einem ersten Längsrand, einem zweiten Längsrand umfaßt, wobei der genannte absorbierende Artikel (22) dadurch gekennzeichnet ist, daß er weiters umfaßt:
einen ersten und einen zweiten Rückhaltebauteil (78) mit einem nach innen gerichteten Längsrand und einem nach außen gerichteten Längsrand (78a), wobei der genannte nach innen gerichtete Längsrand mit der genannten Kleidungsstückseite des genannten absorbierenden Artikels (20) verbunden ist, die genannten Rückhaltebauteile (78) an Verbindungspunkten (72) einwärts vom Längsrand des genannten absorbierenden Aufbaus verbunden sind, um vertiefte Zonen (68) zu bilden, wobei die genannten Verbindungspunkte (72) sich entlang dem genannten nach innen gerichteten Längsrand der genannten Rückhaltebauteile erstrecken,
der genannte erste und der genannte zweite Lappen (24) sich seitlich auswärts vom genannten Hauptkörperabschnitt (22) erstrecken, wobei die genannten Lappen (24) einen proximalen Rand (32) anliegend an eine Verbindungslinie (30), wo sie mit dem genannten Hauptkörperabschnitt (22) verbunden sind, und einen distalen Rand (34), welcher weg von der Verbindungslinie (30) angeordnet ist, aufweisen und die genannten Lappen einen Lappenfixierungsbauteil (56) daran befestigt aufweisen und mindestens ein Abschnitt der genannten Lappen in die genannte vertiefte Zone (68) weggesteckt ist.

2. Der absorbierende Artikel (20) nach Anspruch 1, bei welchem der genannte Verbindungspunkt (72) des genannten Rückhaltebauteils (78) sich 0,6 cm bis 2,5 cm (ein Viertel Inch bis ein Inch), vorzugsweise 2 cm (drei Viertel eines Inch), einwärts vom genannten Längsrand des genannten absorbierenden Aufbaus befindet.

3. Der absorbierende Artikel (20) nach Anspruch 1, bei welchem der genannte Lappen (24) einen ergreifbaren Laschenbauteil (90) umfaßt, welcher sich seitlich auswärts über den nach außen gerichteten Längsrand (78a) des genannten Rückhaltebauteils (78) hinaus erstreckt.

4. Der absorbierende Artikel (20) nach Anspruch 1, bei welchem der genannte Lappen (24) mit dem genannten Hauptkörperabschnitt (22) verbunden ist.

5. Der absorbierende Artikel (20) nach Anspruch 1, welcher weiters einen Mittelkissenfixierungsbauteil umfaßt, welcher einen Klebstoff aufweist, welcher mit mindestens einem Abschnitt der genannten Rückhaltebauteile (78) verbunden ist.

6. Der absorbierende Artikel 20 nach Anspruch 1, welcher weiters einen Mittelkissenfixierungsbauteil umfaßt, welcher ein mechanisches Befestigungssystem aufweist.

7. Der absorbierende Artikel (20) nach Anspruch 1, bei welchem der genannte erste Lappen (24) und der genannte zweite Lappen (24) jeweils eine Lappenquermittellinie (T₁) umfassen, welche die Hauptlängsmittellinie (L) des absorbierenden Artikels (20) schneidet und den absorbierenden Artikel (20) in vier Viertel unterteilt, wobei jedes der genannten Viertel eine Zone von unterschiedlicher Verlängerbarkeit umfaßt.

## Revendications

1. Article absorbant (20) destiné à être porté dans un sous-vêtement (11) d'une utilisatrice, ledit article absorbant (20) présentant un côté faisant face au corps et un côté de sous-vêtement, ledit article absorbant (20) comprenant une partie de corps principal (22), un premier rabat (24) et un deuxième rabat (24), ladite partie de corps principal (22) ayant une ligne médiane longitudinale L, ladite partie de corps principal (22) comprenant un ensemble absorbant présentant un premier bord longitudinal, un deuxième bord longitudinal, ledit article absorbant (22) étant caractérisé en ce qu'il comprend, en outre :
un premier et un deuxième élément de retenue (78) présentant un bord intérieur longitudinal et un bord extérieur longitudinal (78a), ledit bord intérieur longitudinal étant réuni audit côté de sous-vêtement dudit article absorbant (20), lesdits éléments de retenue (78) étant réunis en des points de raccordement (72) à l'intérieur du bord longitudinal dudit ensemble absorbant pour former des surfaces évidées (68), lesdits points de raccordement (72) s'étendant le long dudit bord intérieur longitudinal dudit élément de retenue, et lesdits premier et deuxième rabats (24) s'étendent latéralement vers l'extérieur depuis ladite partie de corps principal (22) dans laquelle lesdits rabats (24) présentent un bord proximal (32) adjacent à une ligne de jonction (30) où ils sont réunis à ladite partie de corps principal (22), et un bord distal (34) placé à distance de la ligne de jonction (30), et lesdits rabats comprennent un élément d'assujettissement de rabat (56) réuni à ceux-ci, et au moins une partie desdits rabats est repliée dans ladite surface évidée (68).

2. Article absorbant (20) selon la revendication 1, dans lequel ledit point de raccordement (72) dudit élément de retenue (78) a 0,6 cm à 2,5 cm (un quart de pouce à un pouce), de préférence 2 cm (trois quarts de pouce), à l'intérieur dudit bord longitudinal dudit ensemble absorbant.

3. Article absorbant (20) selon la revendication 1, dans lequel ledit rabat (24) comprend un élément formant patte saisissable (90) qui s'étend latéralement vers l'extérieur au-delà du bord extérieur longitudinal (78a) dudit élément de retenue (78).

4. Article absorbant (20) selon la revendication 1, dans lequel ledit rabat (24) est réuni à ladite partie de corps principal (22).

5. Article absorbant (20) selon la revendication 1, comprenant, en outre, un élément central d'assujettissement de tampon qui comprend un adhésif qui est réuni à au moins une partie desdits éléments de retenue (78).

6. Article absorbant (20) selon la revendication 1, comprenant, en outre, un élément central d'assujettissement de tampon qui comprend un système de fixation mécanique.

7. Article absorbant (20) selon la revendication 1, dans lequel ledit premier rabat (24) et ledit deuxième rabat (24) comprennent chacun une ligne médiane transversale de rabat (T₁) qui rencontre la ligne médiane longitudinale principale (L) de l'article absorbant (20) et divise l'article absorbant (20) en quatre quarts, chacun desdits quatre quarts comprenant une zone d'extensibilité différentielle.
